# EUROPEAN PATENT APPLICATION

(11) **EP 3 459 566 A1**
(43) Date of publication of application: **27.03.2019**
(21) Application number: 17192863.3
(22) Date of filing: 25.09.2017
(51) Int. Cl.: A61K 47/00, C12P 19/04

(54) **METHOD FOR OBTAINING A SELF-SUPPORTING BACTERIAL FOAM AND THE FOAM OBTAINED BY SAID METHOD**

(71) Applicant: ETH Zurich, 8092 Zurich (CH)
(72) Inventor: FISCHER, Peter, 8032 Zürich (CH); RÜHS, Patrick, 8050 Zürich (CH); LOPEZ GOMEZ, Yuly Andrea, 6005 Luzern (CH)

(57) **Abstract**

The present invention relates to a method for obtaining a self-supporting bacterial foam, the method comprising the steps of: providing a growth medium for supporting bacterial growth; adding at least one viscosifying agent to the bacterial growth medium and solubilizing the at least one viscosifying agent; adding at least one foaming agent to the mixture of bacterial growth medium and viscosifying agent; heating the mixture of bacterial growth medium, viscosifying agent and foaming agent for solubilizing the at least one foaming agent; after cooling inoculating the mixture with at least one biofilm / solid matrix producing bacteria; introducing air bubbles into the mixture, and cultivating the bacterial culture until a stable self-supporting bacterial foam is formed

## Description

The present invention relates to a method for obtaining a self-supporting bacterial foam, a bacterial foam obtained by this method and a 3D scaffold comprising said bacterial foam.

### Description

Bacteria survive and thrive in diverse ecological niches from hot springs to cold glaciers due to their ability to adapt their metabolism to their environment. To protect themselves from such harsh living conditions, bacteria form biofilms from a diverse selection of biopolymers.

Bacterial cellulose, an extracellular biopolymer produced by various species of bacteria, is very biocompatible for eukaryotic cell growth (H. Backdahl et al., Mechanical properties of bacterial cellulose and interactions with smooth muscle cells. Biomaterials. 27, 2141-2149, 2006), has a strong water retention, and superior mechanical strength. These properties are used in the biomedical industry for the production of artificial skin or medical patches as well as in food products (I. Sulaeva, U. Henniges, T. Rosenau, A. Potthast, Bacterial cellulose as a material for wound treatment: Properties and modifications. A review. Biotechnol. Adv. 33, 1547-1571, 2015; W. K. Czaja, D. J. Young, M. Kawecki, R. M. Brown, The Future Prospects of Microbial Cellulose in Biomedical Applications. Biomacromolecules. 8, 1-12, 2007).

Microporous bacterial cellulose may be used as a potential scaffold for bone regeneration. However, for bone applications a microscopic pore structure is needed to facilitate osteoblast ingrowth and formation of mineralized tissue. In one approach such pore structures were introduced into the bacterial cellulose by incorporating 300-500 µm paraffin wax microspheres into the fermentation process. The paraffin microspheres were subsequently removed (Zaborowska et al., Acta Biomaterialia, 2010, 6, 2540-2547).

In another approach bacterial cellulose with a porous structure was prepared via a facile surfactant-assisted foaming method in azodicarbonamide (AC) -NaOH solution. The procedure comprises immersing and foaming bacterial cellulose membranes in AC solution, washing and subsequent freeze drying (Yin et al., Material Letters, 2012, 81, 131-134).

Thus, the methods for obtaining porous bacterial cellulose known so far require additional auxiliary means for generating pores in a growing bacterial culture or the pores are introduced into a previously generated bacterial cellulose membrane.

The object of the present invention was to overcome these drawbacks. It was in particular a goal to provide a method that enables to: a) tune cellulose porosity for e.g. biomedical, cosmetic, thermal or food applications; b) create food structures mimicking plant cell shape; and c) avoid tedious extraction process of bacterial cellulose.

This object is solved by a method for obtaining a self-supporting bacterial foam according to claim 1, a bacterial foam obtained by this method according to claim 14 and a 3D scaffold comprising said bacterial foam according to claim 15.

Accordingly, a method for obtaining a self-supporting bacterial foam is provided, the method comprising the steps of:
- providing a growth medium for supporting bacterial growth;
- adding at least one viscosifying agent to the bacterial growth medium and solubilizing the at least one viscosifying agent;
- adding at least one foaming agent to the mixture of bacterial growth medium and viscosifying agent;
- heating the mixture of bacterial growth medium, viscosifying agent and foaming agent for solubilizing the at least one foaming agent;
- after cooling inoculating the mixture with at least one biofilm and/or solid matrix producing bacteria;
- introducing air bubbles into the mixture, and
- cultivating the bacterial culture until a stable self-supporting bacterial foam is formed.

The present method uses stirred and aerated bacterial cultures for improving the productivity. By increasing the total contact area to oxygen in form of surfactant stabilized air bubbles (foam templating), (a) the yield of solid matrix (such as cellulose) production is increased and (b) a foam structure made of cellulose or other biomaterials is created. By tuning the bubble size distribution, it is possible to change the porosity (of the intrinsically porous cellulose) and air integration into the newly created aerogel or foam.

The initial foam template is formed by using bacteria, bacterial media, and adding a specific surfactant. As a consequence of bacterial growth and mobility towards oxygen, bacterial cellulose is formed at the interface between growth media and air. The colonization of the interface by the bacteria result in a cellulose foam structure in which the air bubble size can be directly determined by the initial bubble size, thus creating bubbles with defined size.

In comparison to traditional methods the use of prefabricated foam as template for bacteria growth and cellulose production has the following advantages over other bacterial foams: Less processing steps; Native cellulose structure; No need for chemical or mechanical treatment prior application; Bubble size determine the porosity of the foam which enables specific cell growth.

In an embodiment of the present method the self-supporting bacterial foam comprises on one of the following: bacterial cellulose, bacterial calcium carbonate or bacterial amyloid, in particular bacterial cellulose.

In a preferred embodiment of the present method the at least one biofilm / solid matrix producing bacteria is selected from cellulose producing Gram-negative bacteria species such as *Acetobacter, Azotobacter, Rhizobium, Pseudomonas, Salmonella, Alcaligenes,* and Gram-positive bacteria species such as *Sarcina ventriculi.*

In certain of these embodiments, the cellulose producing bacteria is selected from *Acetobacter xylinum, Gluconacetobacter hansenii, Gluconacetobacter xylinus, Acetobacter sp., Sporosarcina pasteurii, Deleya halophile and Proteus mirabilis,* particularly selected from *A. xylinum* strains BRC5, BPR2001, E25 or NUST4.1, G. hansenii strain PJK, Acetobacter sp. strains V6 or A9, *G. xylinus* strains K3, IFO 13773 and RKY5, *Sporosarcina pasteurii* ATCC 11859. The most preferred cellulose producing bacteria is a strain of *Acetobacter xylinum.*

It is also conceivable to use genetically modified organisms.

In another embodiment of the present method the at least one biofilm / solid matrix producing bacteria is selected from a group of calcium carbonate producing bacteria comprising *Magnetospirillum gryphiswaldense* MSR-1, *Magnetospirillum magneticum* AMB-1, *Magnetospirillum magneticum* MGT-1, *Magnetovibrio* MV-1, *Magnetococcus* sp. MC-1, *Magnetospirillum* sp. WM-1 *Magnetospirillum magnetotacticum* MS-1 and *Desulfovibrio magneticus* RS-1.

In a further variant of the present method the bacterial growth media comprises a complex media or a minimal media.

A complex media as an undefined medium typically contains a carbon source such as glucose, water, various salts, a source of amino acids and nitrogen (e.g., beef, yeast extract, wherein the amino-acid source contains a variety of compounds with the exact composition being unknown).

A minimal medium typically contains water, a carbon source, which may be a sugar such as glucose, or a less energy-rich source such as succinate; various salts, which may vary among bacteria species and growing conditions. These salts generally provide essential elements such as magnesium, nitrogen, phosphorus, and sulfur to allow the bacteria to synthesize protein and nucleic acids.

In a further preferred embodiment of the present method the at least one viscosifying agent is a polysaccharide capable of forming a viscous solution. Thus, the term "viscosifying agent" in the context of the present specification relates to a polysaccharide capable of increasing the viscosity in the aqueous phase. By increasing the viscosity of the growth media the foam half life is also increased. Therefore, the viscosifying effect is essential for foam formation. Besides, with the addition of a viscosifying agent, the bacterial media is viscosified to prevent water drainage.

The at least one viscosifying agent is a polysaccharide selected from the group comprising alginate, alginate sulfate, gellan sulfate, starch, modified starch, gelatin, pectin, cellulose, bacterial cellulose, chitosan, gellan gum, acylated and/or sulfated gellan gum, guar gum, cassia gum, konjac gum, Arabic gum, ghatti gum, locust bean gum, xanthan gum, xanthan gum sulfate, carrageen, carrageen sulfate, and a mixture of any of the gel forming polysaccharides mentioned previously; xanthan being the most preferred polysaccharide

The at least one viscosifying agent is added to the bacterial growth medium in a concentration of at least 0.1 wt%, preferably at least 0.5 wt%, more preferably at least 1.0 wt%. The concentration range may be between 0.1 and 3.0 wt%, preferably between 0.5 and 1,5 wt%. Typical concentrations of the viscosifying agent are 0.1 wt%, 0.5 wt%, 1 wt% and 1.5 wt%.

The viscosity of the growth media mixture may be adjusted to a value between 1 and 50 Pas, preferably 5 and 30 Pas, in particular preferably 10-20 Pas. A typical viscosity range may be between 1-10 Pas.

In another preferred embodiment of the present method the at least one foaming agent is selected from the group comprising fatty acids, proteins and surfactants. Important to note is that the suitable foaming agent should show no inhibitory effect on bacterial growth.

Suitable foaming agents are: fatty acids such as cremodan (cremodan 30 is a stabilizer used typically in ice cream and sorbet), proteins such as whey protein isolate, and/or surfactants such as Tween 20 or sodium stearoyl-2-lactylate (SSL). Cremodan is the most preferred foaming agent.

The at least one foaming agent is added to the bacterial growth medium in a concentration of at least 1wt%, preferably at least 2 wt%, more preferably at least 3 wt%. The concentration range of the foaming agent may be between 1 and 5 wt%, preferably between 2 and 3 wt%.

In a more preferred variant of the present method the at least one viscosifying agent is added in a concentration of at least 1 wt% and the at least one foaming agent is added in a concentration of at least 3 wt%.

In an even more preferred variant xanthan is used as viscosifying agent in a concentration between 0.1 and 1.5 wt% and Cremodan is used as foaming agent in a concentration between 1 and 4 wt%. In a most preferred embodiment the bacterial growth medium comprises 1 wt % Xanthan and 3 wt% Cremodan.

It is furthermore preferred, if the bubbles are introduced into the growth media by means of conventional mixtures. Such conventional mixtures may be static mixers, rotating membrane systems, bubbling techniques, extrusion based or whipping devices.

The size of the formed bubbles is between 10 and 500 µm, preferably between 50 and 300 µm, most preferably between 50 and 200 µm. In order to obtain a stable foam, the mixing time with the conventional mixer may be at least 3 minutes. With 3 min, bubbles in the size of 50 - 300 µm are formed. With longer mixing times, smaller bubbles are formed with distributions from 50 - 200 µm.

The method according to the invention enables the production of a self-supporting bacterial foam with a pore size between 10 and 500 µm, preferably between 50 and 300 µm, most preferably between 50 and 200 µm.

Such self-supporting bacterial foams, in particular bacterial cellulose foams, may be used as a 3D scaffold for biomedical applications, such as for wound dressing, breast implants, and envelopes for implants. Further applications may comprise food and cosmetics.

The invention is further explained in detail by means of the following examples with references to the figures. It shows:
- Figure 1 A-F: schematic overview of biofilm formation at water-air interfaces to form 3D bacterial cellulose scaffolds;
- Figure 2 A-B: images of stabilized bacterial cellulose foam after 50 h (A) and images of foam structure at different scales (B);
- Figure 3 A-B: Diagrams illustrating (A) Overrun and (B) foam stability of foam formed by using different concentrations of Cremodan as foaming agnet in bacterial media;
- Figure 4: Diagram illustrating the foam stability of bacterial media with Cremodan as foaming agent with and without *A*. *xylinum*; and
- Figure 5 A-B: Diagrams illustrating (A) Overrun and (B) foam stability of a foam with 3 wt% Cremodan and varying xanthan concentrations (0.1 wt%, 0.5 wt%, 1 wt%, and 1.5 wt%).

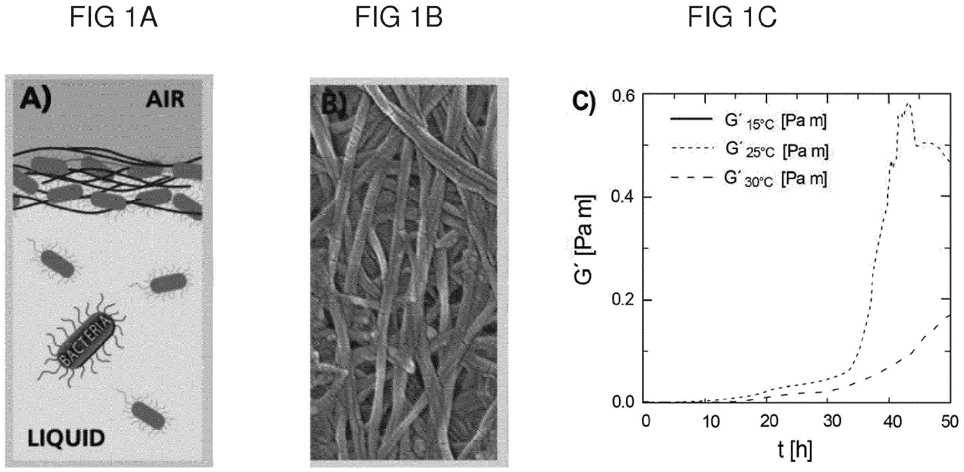

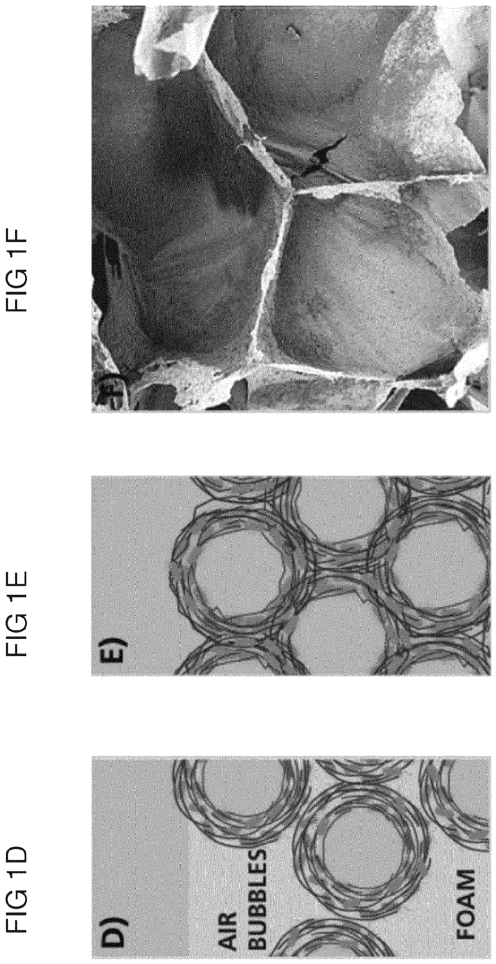

The bacteria considered here, *A. xylinum,* produces large quantities of nano-cellulose fibers at the water-air interface (Figure 1A) forming a pellicle with high mechanical properties (Figure 1 B; Microscopy image of the interwoven bacterial cellulose fibers). The biofilm formation at the water-air interface is monitored with interfacial rheology to identify the ideal biofilm growing conditions such as pH and temperature.

For bacterial biofilm formation, 1 wt% of a fully grown *A. xylinum* ATCC-700178 culture was grown in bacterial media (mannitol 25 g/l, yeast extract 5 g/l, peptone 3 g/l in water) for 50 hours in an interfacial rheological setup.

The bacterial growth and biofilm formation were measured by an oscillatory time sweep (strain = 0.1 %, frequency = 1 rad/s) at 15, 25, and 30°C monitoring the elastic modulus G', equivalent to biofilm components present at the interface, biofilm thickness, and mechanical integrity (Figure 1 C). An increase of G' is observed after 25 to 35 hours at 25°C, showing that a biofilm is formed. At 15 and 30°C biofilm formation is slower, visible by the slow increase of elasticity when compared to the elastic modulus increase at 25°C.

As observed in Figure 1B, the cellulose production is directly limited to the interface forming a mat of interwoven cellulose fibers.

So far, the water-air surface dependency of biofilm growth has been used to form surface-patterned implants, ear transplants, and potential blood vessels by using oxygen permeable silicon moulds. Additionally, the ability of the bacteria to form biofilms at the water-air interface has been exploited, to form macroporous foams by using wax and starch particles as templates.

The method according to the invention uses a foam templating approach exploiting the biofilm formation at water-air interfaces to form 3D bacterial cellulose scaffolds (Figure 1D - F).This is achieved by foaming the bacteria media with an overrun of 200% and therefore increasing the total surface area of bacterial contact to the air phase by about 800 times in comparison to the flat water-air interface. In order to achieve sufficient foam stability of the bacteria media, xanthan gum (Jungbunzlauer AG, Austria) is added as a thickener (or viscosifying agent) at 0.5 wt% to increase the foam half-life and Cremodan at 3 wt% (fatty acids, Super MB Danisco) to stabilize the air bubbles.

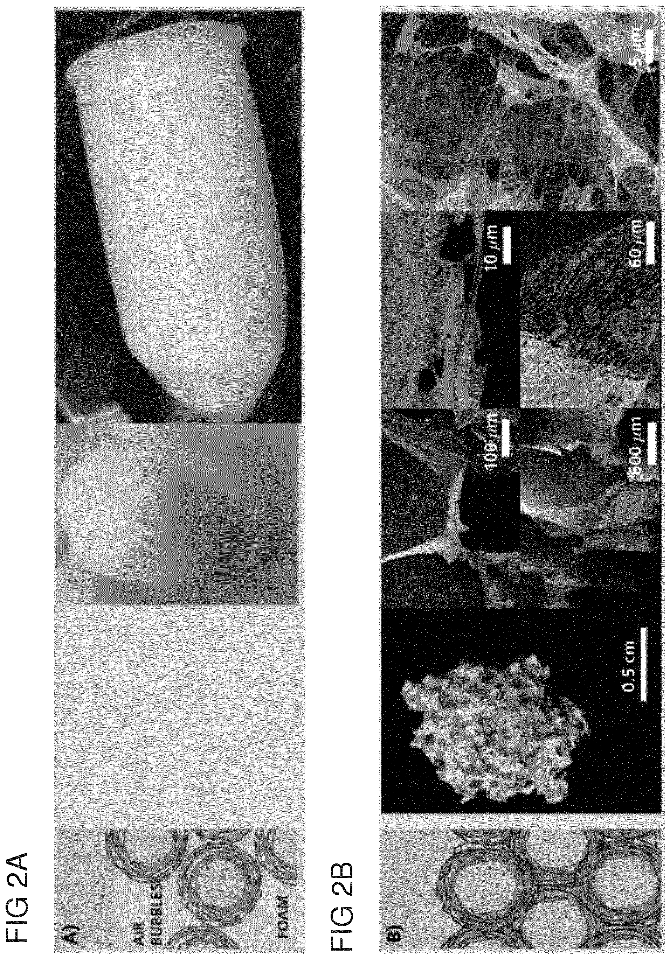

First, xanthan and Cremodan were added to the bacterial media and heated to 80°C while stirring to dissolve both components. After cooling, the bacteria were inoculated at 1%, and the modified media foamed with a standard cappuccino mixer to form droplets around 150 µm. The air bubbles are stabilized after 2 days through bacterial cellulose formation and form a complete cellulose network after 6 days. With this technique, a self-supporting cellulose foam was successfully engineered without any additional processing steps (Figure 2A).

For biomedical applications, the foam can be washed in NaOH at 80°C to remove all bacterial cells and sterilized without a loss of integrity. By tuning the bubble size distribution, it is suggested that the porosity (of the intrinsically porous cellulose) and air integration into the newly created aerogel or foam can be tuned for example to meet the stringent criteria of cell proliferation and differentiation present in tissue engineering applications. Subsequent drying or freeze drying leads to the formation of light porous structures of cellulose (Figure 2B). As visible through SEM, the dried foam forms hexagonal structures with cellulose lamella in between the macroscopic pores. Inside of the material cellulose layers are formed with an additional level of porosity due to the fibrous nature of the cellulose. By freeze drying the foam structure, an additional effect can be observed, forming a super porous dried foam with a defined freezing direction.

### a) Media for bacterial growth and incompatibility concerns with foamability

Bacteria were grown in the following complex media composition:

**Table 1: complex media composition for growth of A. xylinum**

| **Composition** | **Amount (g/L)** |
|---|---|
| Yeast extract | 5.0 |
| Peptone | 3.0 |
| Mannitol | 25.0 |

However, based on the amount of possible interactions of foaming agents and media components, any kind of culture media where these bacteria can be grown, such as the glucose based media listed in Table 2:

**Table 2: Alternative glucose based media for growth of A. xylinum**

| **Medium component** | **Amount/L** |
|---|---|
| D-Glucose C₆H₁₂O₆ | 20 g |
| Diazanium Sulfate (Ammonium Sulfate) (NH₄)₂SO₄ | 3.5 g |
| Potassium KH₂PO₄ Dihydrogen Phosphate (Phosphoric Acid) | 7 g |
| Sodium Phosphate Dibasic Dodecahydrate | 3.4 g |
| (Na₂HPO₄)₁₂H₂O | |
| Magnesium Sulfate Heptahydrate (MgSO₄)₇H₂O | 2.1 g |
| Boric Acid H₃BO₃ | 4.3 mg |
| Ferrous Sulfate Heptahydrate (FeSO₄)₇H₂O | 9.5 mg |
| Pyridine-3-Carboxamide (Nicotinamide) C₆H₆N₂O | 0.7 mg |
| Ethanol C₂H₅OH | 6 mL |

Media components can contain any number of proteins and salts as long as a sugar source or a modified sugar source is included which is necessary for the growth of cellulose. The pH in this case is irrelevant, although for growth of bacteria a pH between 5-7 is optimal.

### b) Concentration of Cremodan as foaming agent and Xanthan as viscosifying agent

The concentrations of Cremodan (foaming agent) and xanthan (viscosifying agent) necessary to obtain a stable foam were determined.

In a first set of experiments varying concentrations of Cremodan (1, 2, 3, and 4 wt%) were tested and bacterial media was foamed accordingly. The overrun (ratio between original volume of the liquid phase and before foaming and foam volume after foaming) as a parameter for the foam ability was determined

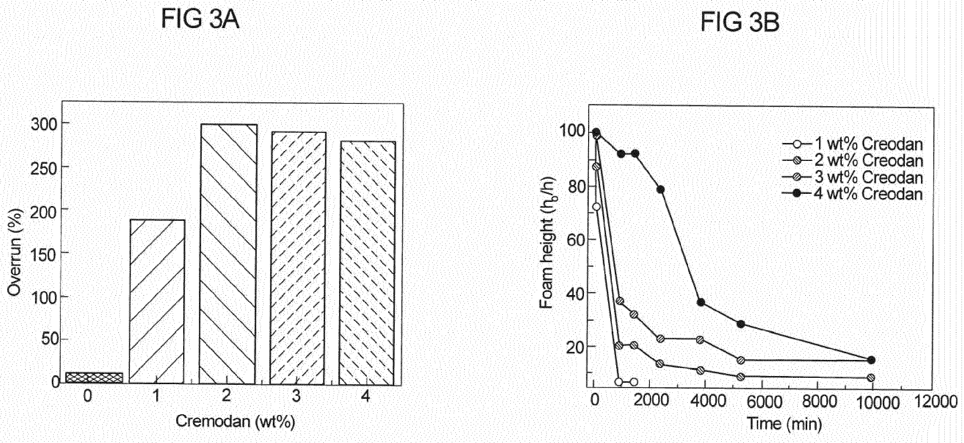

(Fig. 3A) whereas the foam height displays the foam stability (Fig. 3B). By using Cremodan alone as an emulsifying agent, a stable foam can be formed at a minimum concentration of 2 wt% (Figure 3B). With higher Cremodan concentrations, a stable foam is formed.

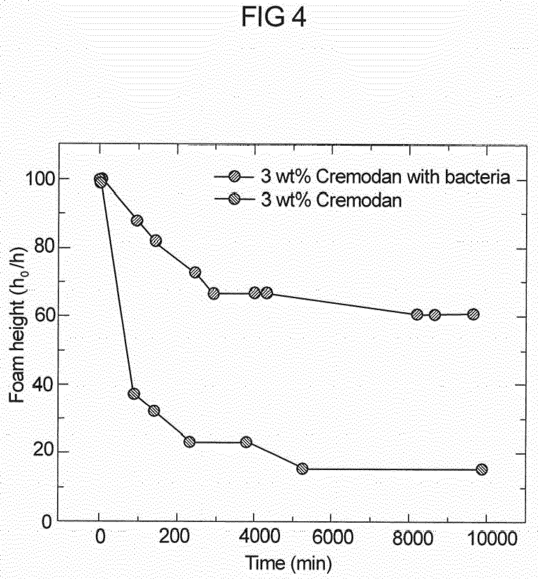

However, without using a viscosifying agent, water drainage causes water loss in the foam destabilizing the foam structure. The formation of bacterial cellulose during bacterial growth stabilizes the foam. Even though bacterial growth prevents foam height loss through cellulose formation, water drainage leads to a loss of water out of the foam (Figure 4).

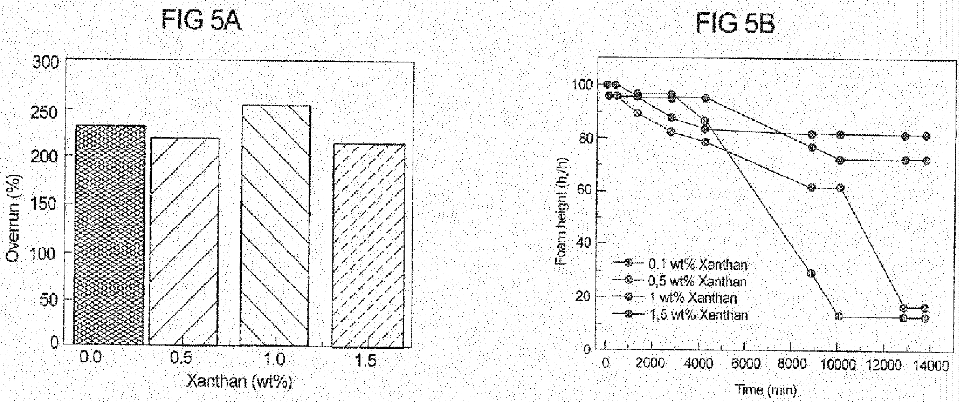

By adding of xanthan as viscosifying agent, the bacterial media is viscosified to prevent water drainage (Figure 5A). A foam can be formed at all concentration from 0.1 - 1.5 wt% corresponding to a viscosity increase from 0.001 Pas (water) up to 100 Pas (at a shear rate of 0.001 s⁻¹). Starting at a concentration of 0.2 wt% enough viscosity prevents water drainage and therefore contributes to foam stability (Figure 5B).

In summary, a concentration of at least 3 wt% Cremodan and 0.2 wt% xanthan is necessary to form a foam which is stable until bacterial cellulose growth leads to further foam stabilization.

### c) Foam formation:

In order to obtain a stable foam, the mixing time with the conventional mixer is at least 3 minutes. With 3 min, bubbles in the size of 50 - 300 µm are formed. With longer mixing times, smaller bubbles are formed with distributions from 50 - 200 µm. With stronger mixing techniques smaller bubbles should be possible.

## Claims

1. Method for obtaining a self-supporting bacterial foam, the method comprising the steps of:
- providing a growth medium for supporting bacterial growth;
- adding at least one viscosifying agent to the bacterial growth medium and solubilizing the at least one viscosifying agent;
- adding at least one foaming agent to the mixture of bacterial growth medium and viscosifying agent;
- heating the mixture of bacterial growth medium, viscosifying agent and foaming agent for solubilizing the at least one foaming agent;
- after cooling inoculating the mixture with at least one biofilm / solid matrix producing bacteria;
- introducing air bubbles into the mixture, and
- cultivating the bacterial culture until a stable self-supporting bacterial foam is formed.

2. Method according to claim 1, **characterized in that** the self-supporting bacterial foam comprises on one of the following: bacterial cellulose, bacterial calcium carbonate or bacterial amyloid, in particular bacterial cellulose.

3. Method according to one of the claims 1 or 2, **characterized in that** the at least one biofilm / solid matrix producing bacteria is selected from cellulose producing Gram-negative bacteria species such as *Acetobacter, Azotobacter, Rhizobium, Pseudomonas, Salmonella, Alcaligenes,* and Gram-positive bacteria species such as *Sarcina ventriculi* and *Bacillus subtilis.*

4. Method according to one of the claims 1 or 2, **characterized in that** the at least one biofilm / solid matrix producing bacteria is selected from a group of calcium carbonate producing bacteria comprising *Magnetospirillum gryphiswaldense* MSR-1, *Magnetospirillum magneticum* AMB-1, *Magnetospirillum magneticum* MGT-1, *Magnetovibrio* MV-1, *Magnetococcus* sp. MC-1, *Magnetospirillum* sp. WM-1 *Magnetospirillum magnetotacticum* MS-1 and *Desulfovibrio magneticus* RS-1.

5. Method according to any of the preceding claims, **characterized in that** the bacterial growth media comprises a complex media or a minimal media.

6. Method according to any of the preceding claims, **characterized in that** the at least one
viscosifying agent is a polysaccharide capable of forming a viscous solution.

7. Method according to any of the preceding claims, **characterized in that** the at least one viscosifying agent is a polysaccharide selected from the group comprising alginate, alginate sulfate, gellan sulfate, starch, modified starch, gelatin, pectin, cellulose, bacterial cellulose, chitosan, gellan gum, acylated and/or sulfated gellan gum, guar gum, cassia gum, konjac gum, Arabic gum, ghatti gum, locust bean gum, xanthan gum, xanthan gum sulfate, carrageen, carrageen sulfate.

8. Method according to any one of the preceding claims, **characterized in that** the at least one viscosifying agent is added to the bacterial growth medium in a concentration of at least 0.1 wt%, preferably at least 0.5 wt%, more preferably at least 1.0 wt%.

9. Method according to any one of the preceding claims, **characterized in that** the at least one foaming agent is selected from the group comprising fatty acids, proteins and surfactants.

10. Method according to any one of the preceding claims, **characterized in that** the at least one foaming agent is added to the bacterial growth medium in a concentration of at least 1wt%, preferably at least 2 wt%, more preferably at least 3 wt%

11. Method according to any one of the preceding claims, **characterized in that** the at least one viscosifying agent is added in a concentration of at least 1 wt% and the at least one foaming agent is added in a concentration of at least 3 wt%.

12. Method according to any one of the preceding claims, **characterized in that** the bubbles are introduced into the growth media by means of conventional mixtures.

13. Method according to any one of the preceding claims, **characterized in that** the size of the formed bubbles is between 10 and 500 µm, preferably between 50 and 300 µm, most preferably between 50 and 200 µm.

14. A self-supporting bacterial foam obtainable by a method according to any one of the preceding claims **characterized by** a pore size between 10 and 500 µm, preferably between 50 and 300 µm, most preferably between 50 and 200 µm.

15. A 3D scaffold comprising a self-supporting bacterial foam according to claim 14 for biomedical applications.
